# EUROPEAN PATENT APPLICATION

(11) **EP 4 245 287 A1**
(43) Date of publication of application: **20.09.2023**
(21) Application number: 22162681.5
(22) Date of filing: 17.03.2022
(51) Int. Cl.: A61K 8/02, D01D 5/00, D04H 1/728, A61Q 19/00

(54) **PROCESS FOR MAKING A SKINCARE PRODUCT**

(71) Applicant: Dacheng Filter Materials Co., Ltd., New Taipei City 236 (TW)
(72) Inventor: YEN, YI-HSUAN, 236 New Taipei City (TW); CHEN, WAN-CHI, 236 New Taipei City (TW)
(74) Representative: Lang, Christian

(57) **Abstract**

A process for making facial masks includes the step of providing a solution of materials comprising at least one moisturizer, the step of making nanometer fibers from the solution of materials, the step of making a skincare product from the nanometers, and the step of packaging the skincare product.

## Description

### BACKGROUND OF INVENTION

### 1. FIELD OF INVENTION

The present invention relates to skincare product and, more particularly, to a process for making a skincare product.

### 2. RELATED PRIOR ART

JP 3215852 discloses a plaster including a layer of cosmetics coated on a carrier. The cosmetics include casein and natural derivatives. The layer of cosmetics can be replaced with a layer of gel that is made by soaking amylose in cosmetic liquid to increase attachability to human skin. However, the cosmetic liquid often includes vitamin C that can easily deteriorate before the cosmetic liquid is absorbed by the human skin. Moreover, the carrier is often made of plant or vegetable fibers such as paper made of wood fibers. The carrier can easily be torn and inadequately flexible or compliant to the human skin.

The present invention is therefore intended to obviate or at least alleviate the problems encountered in the prior art.

### SUMMARY OF INVENTION

It is the primary objective of the present invention to provide a process for making a skincare product that is easily absorbable by human skin and adequately flexible or compliant to the human skin.

To achieve the foregoing objective, the process includes the step of providing a solution of materials comprising at least one moisturizer, the step of making nanometer fibers from the solution of materials, the step of making a skincare product from the nanometers, and the step of packaging the skincare product.

Other objectives, advantages and features of the present invention will be apparent from the following description referring to the attached drawings.

### BRIEF DESCRIPTION OF DRAWINGS

The present invention will be described via detailed illustration of the preferred embodiment referring to the drawings wherein:
FIG. 1 is a flow chart of a process for making a skincare product according to the preferred embodiment of the present invention;
FIG. 2 is a flow chart of a subroutine of the process shown in FIG. 1;
FIG. 3 is a sketch of a fiber-making apparatus for producing nanometer fibers in the subroutine shown in FIG. 2;
FIG. 4 is a front view of a skincare product made in the process shown in FIG. 1; and
FIG. 5 is a side view of the skincare product shown in FIG. 4.

### DETAILED DESCRIPTION OF PREFERRED EMBODIMENT

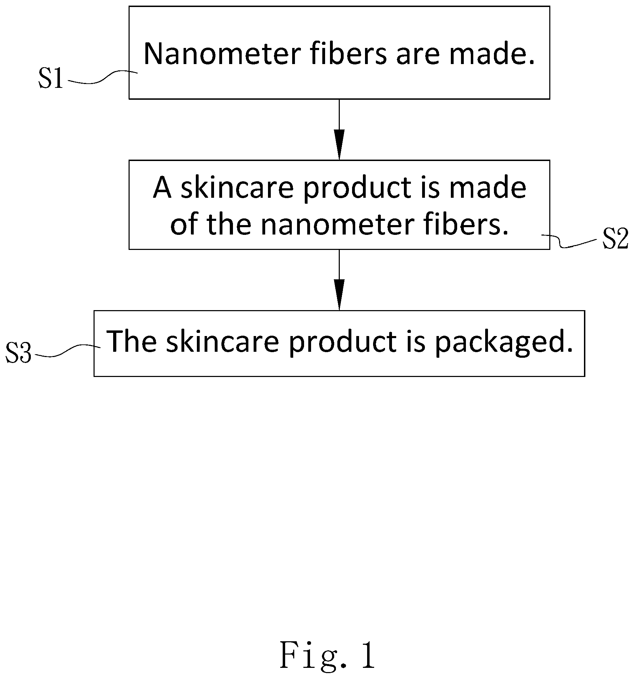

Referring to FIG. 1, a process for making facial masks includes three subroutines S1, S2 and S3 according to the preferred embodiment of the present invention. At subroutine S1, nanometer fibers are made. At subroutine S2, a skincare product 20 is made of the nanometer fibers. At subroutine S3, the skincare product is packaged.

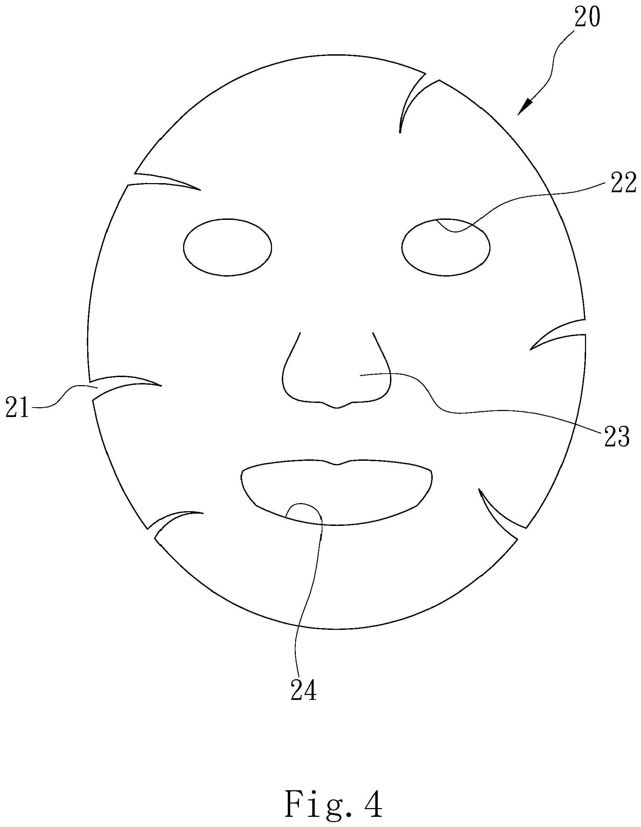

Referring to FIG. 4, skincare product 20 can be a facial mask for example. Skincare product 20 can be a plaster or any other proper product in the form of a film. Skincare product 20 is easily attachable to and then absorbable by the human skin.

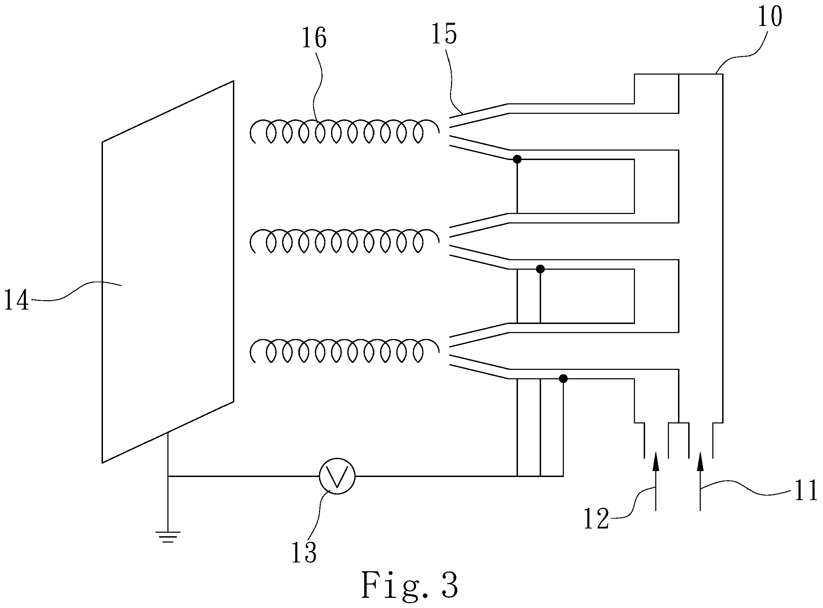

Referring to FIG. 3, skin care product 20 includes materials 11 in the form of nanometer fibers made by a fiber-making apparatus. Thus, materials 11 do not easily deteriorate before they are absorbed by the human skin. Preferably, the fiber-making apparatus is an electrospinning apparatus that includes a feeder 10, a high-voltage power supply 13, a collector 14, and multiple nozzles 15. An electrode of high-voltage power supply 13 is electrically connected to collector 14. Another electrode of high-voltage power supply 13 is electrically connected to nozzles 15.

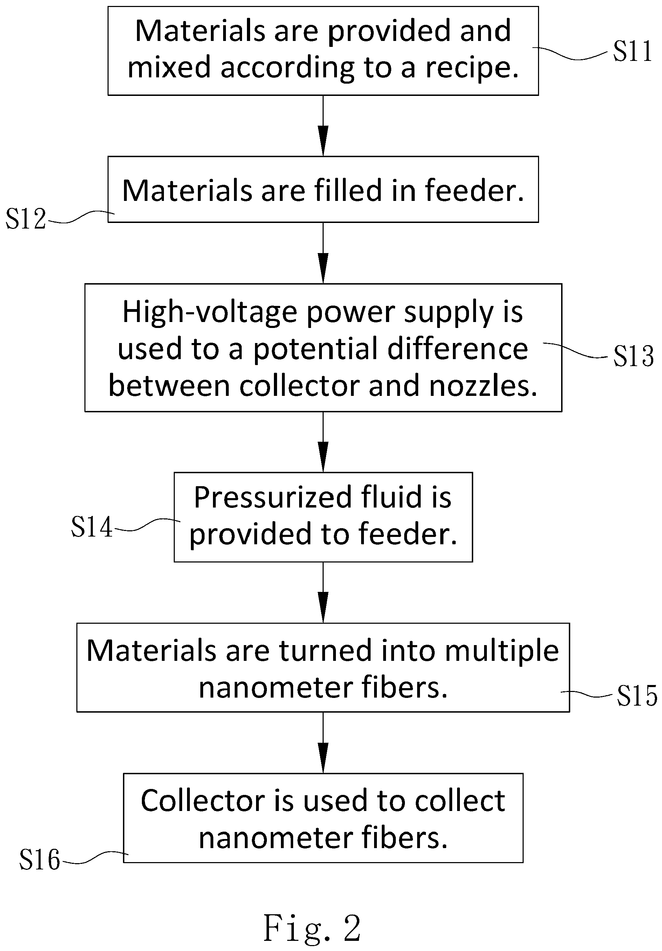

Referring to FIG. 2, subroutine S1 includes several steps.

At S11, materials 11 are provided and mixed according to a recipe. At S12, materials 11 are filled in feeder 10. At S13, high-voltage power supply 13 is used to a potential difference between collector 14 and nozzles 15. At S14, pressurized fluid 12 is provided to feeder 10. Pressurized fluid 12 is preferably pressurized air. At S15, materials are turned into multiple nanometer fibers 16. At S16, collector 14 is used to collect nanometer fibers 16.

At S11, materials 11 include skincare or cosmetic materials and carrier materials. The skincare or cosmetic materials are to be absorbed by the human skin. The carrier materials are used to carry the skincare or cosmetic materials in the form of nanometer fibers 16.

The carrier materials include polymers, antioxidants, natural antibacterial agents, and water for example. The polymers can be physically hydrolyzed or chemically cracked. The polymers can be gelatin, collagen, lignin, soy protein, starch, polylactic acid, chitin, chitosan, polyvinyl alcohol, and/or polycaprolactone.

Where polyvinyl alcohol is used, deionized water is preferably used to dissolve the polyvinyl alcohol. For example, 1 gram of polyvinyl alcohol and 5 ml of water are filled in a jar soaked in hot water at 80°C to 85°C before stirring is executed 100 rpm. Thus, 20% (w/v) polyvinyl alcohol solution is provided. An ultrasonic oscillator is used to dissolve 0.5 gram of gelatin in 5 ml of water. Thus, gelatin solution is provided. Then, the gelatin solution is mixed with the polyvinyl alcohol solution.

Chitosan is a non-toxic natural polymer and a natural antibacterial agent that can be included in the carrier solution to suppress bacteria. Water added with nanometer silver ions is also excellent in suppressing bacteria and hence can be included in the carrier solution.

The skincare materials include quinol, koji acid, vitamin C, and/or any derivatives thereof, tranexamic acid, and/or alpha hydroxy acid. Vitamin C is a water-dissolvable natural antioxidant that is excellent in whitening and aging-resisting. Preferably, 0.1 gram of vitamin C and 0.45 gram of tranexamic acid are included in the carrier solution.

The skincare materials can include hydrophilic moisturizers such as glycerin and propylene glycol into. Preferably, 0.2 ml of glycerin and 0.9 ml of propylene glycol are included in the carrier solution.

At S12, parameters of hardware and/or software of the fiber-making apparatus are set after materials 11 are filled in feeder 10. For example, the diameter of nozzles 15 is set to be 0.5 mm to 2.5 mm. The pressure of pressurized fluid 12, which is preferably pressurized air, is 1 kgf/cm² to 8 kgf/cm². The flow rate of pressurized fluid 12 is 20 l/min to 350 l/min. The voltage of high-voltage power supply 13 is set to provide a DC at 10 kV to 50 kV.

Parameters of the fiber-making apparatus in relation to materials 11 are set to allow the fiber-making apparatus to produce nanometer fibers 16.

For example, 8% of polyvinyl alcohol (PVA) is added to deionized water. The flow rate is set to be 0.36 ml/hr, the voltage is set to be 20.6 kV, and collector 14 is located at 20 cm from nozzles 15.

Alternatively, 12% of polyvinyl alcohol (PVA) is added to deionized water. The flow rate is set to be 0.36 ml/hr, the voltage is set to be 20.6 kV, and collector 14 is located at 20 cm from nozzles 15.

Alternatively, 10% of polyvinyl alcohol (PVA) and 1% of gelatin is added to deionized water. The flow rate is set to be 0.6 ml/hr, the voltage is set to be 16.8 kV, and collector 14 is located at 20 cm from nozzles 15.

Alternatively, 10% of polyvinyl alcohol (PVA) and 5% of gelatin are added to deionized water. The flow rate is set to be 0.6 ml/hr, the voltage is set to be 16.8 kV, and collector 14 is located at 20 cm from nozzles 15.

At S13, the high-voltage power supply 13 is used to create a potential difference between collector 14 and nozzles 15 to facilitate movement of materials 11 in the form of nanometer fibers 16 to collector 14 from nozzles 15. Preferably, positive charge is accumulated at tips of nozzles 15 while negative charges is accumulated on a face of collector 14.

At S14, pressurized fluid 12 is provided to feeder 10. Then, pressurized fluid 12 goes to nozzles 15 through feeder 10. During the travel to nozzle 15, pressurized fluid 12 moves materials 11 to nozzles 15.

At S15, nanometer fibers 16 are sprayed from nozzles 15. At least some of the positive charge is transferred to materials 11 from the tips of nozzles 15 as materials 11 travels past the tips of nozzles 15. The positive charge that remains at the tips of nozzles 15 repels the positive charge transferred to materials 11. Thus, the solution of materials 11 is turned into a cone from a sphere. The cone is called the "Taylor Cone." The surface tension of the Taylor Cone yields to the electric force so that the solution of materials 11 is moved toward collector 14 in the form of a jet from each nozzle 15. In the cone-jet phase, the solvent, i.e., the deionized water is vaporized. Thus, materials 11 are turned into nanometer fibers 16 with a diameter of 100 nm to 1000 nm.

At S16, nanometer fibers 16 are collected by collector 14. Collector 14 is preferably a metal plate. Nanometer fibers 16 are bent or spiral fibers on collector 14.

In another embodiment, collector 14 is a roller in rotation. Thus, a large amount of nanometer fibers 16 can be collected on the surface of the roller in a short period of time.

In another embodiment, two parallel planar electrodes are used to collect oriented nanometer fibers.

In another embodiment, collector 14 can be in the form of a disc, ring or tank.

Alternatively, 10% of polyvinyl alcohol (PVA) and 5% of gelatin are added to the solution of materials 11, the voltage is set to 20 kV, the flow rate is set to be 1.2 ml/hr, and collector 14 is located at 25 cm from nozzles 15. Thus, the diameter of resultant nanometer fibers 16 is 600 nm to 850 nm.

FTIR analysis is executed for superficial function groups. The spectrum shows vitamin C and tranexamic acid. It is proven that materials 11 exist in nanometer fibers 16. Hence, skincare product 20 made of nanometer fibers 16 is moisturizing.

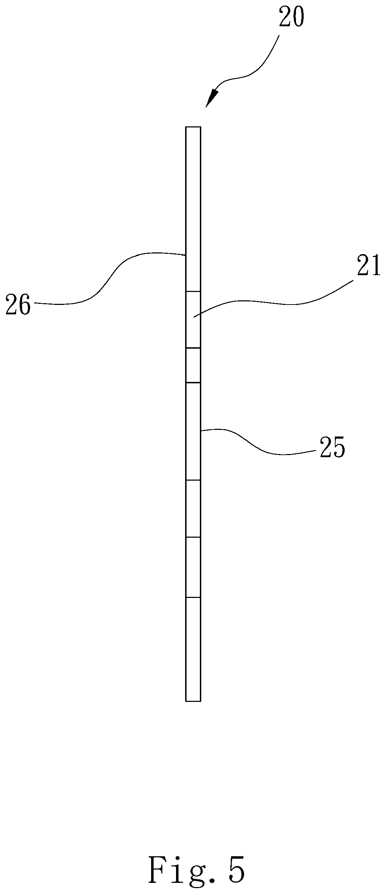

Referring to FIGS. 1, 4 and 5, at S2, skincare product 20 is made of nanometer fibers 16. To make skincare product 20, a film is made of nanometers 16 on a plate for example. Then, a mold is used to cut the film of nanometer fibers 16. Thus, skincare product 20 is made. Skincare product 20 is preferably a facial mask with multiple slits 21 at an edge, two windows 22 corresponding to human eyes, a flip 23 corresponding to a human nose, and an opening 24 corresponding to a human mouth, an internal side 25, and an external side 26.

In another embodiment, another mold can be used to cut skincare product 20 into another shape such as a plaster suitable for use on a user's nose, face or limbs.

At S3, skincare product 20 is packaged. Preferably, vacuum packaging used to package skincare product 20 in a bag or package (not shown). Thus, materials 11, which include the moisturizers, are kept in nanometer fibers 16. That is, materials 11 cannot be vaporized.

Before use, the bag is opened to allow skincare product 20 to be taken from the bag. In use, skincare product 20 is laid on a user's face. Slits 21 render skincare product 20 adequately compliant to the user's face. Thus, internal side 25 of skincare product 20 is in proper contact with the user's face. Windows 22 allow the user to see through skincare product 20. Flip 23 flips from the remaining portion of skincare product 20 and covers the user's nose while allowing the user to breathe. Opening 24 allows the user to eat, drink, and talk. The whitening materials of skincare product 20 are absorbed by the user's skin within 30 min. Accumulated rates of release of the vitamin C and tranexamic acid reach 60% and 20%.

Preferably, proper portions of the skincare materials included in skincare product 20 are absorbed by the user's skin. The carrier materials can easily be washed or wiped away.

The present invention has been described via the illustration of the preferred embodiment. Those skilled in the art can derive variations from the preferred embodiment without departing from the scope of the present invention. Therefore, the preferred embodiment shall not limit the scope of the present invention defined in the claims.

## Claims

1. A process for making facial masks comprising the steps of:
providing a solution of materials comprising at least one moisturizer;
making nanometer fibers from the solution of materials;
making a skincare product from the nanometers; and
packaging the skincare product.

2. The process according to claim 1, wherein the moisturizer comprises a hydrophilic moisturizer.

3. The process according to claim 2, wherein the hydrophilic moisturizer is selected from the group consisting of glycerin and propylene glycol.

4. The process according to claim 1, wherein the solution of materials comprises at least one material selected from the group consisting of gelatin, collagen, lignin, soy protein, starch, polylactic acid, chitin, chitosan, polyvinyl alcohol, polycaprolactone, quinol, koji acid, vitamin C, any derivatives thereof, tranexamic acid, alpha hydroxy acid, and water.

5. The process according to claim 1, wherein the step of providing a solution of materials comprises the steps of:
providing skincare materials;
providing carrier materials; and
mixing the skincare materials, the carrier materials and the moisturizer with one another.

6. The process according to claim 1, wherein the skincare product is selected from the group consisting of a facial mask and a plaster.

7. The process according to claim 1, wherein the step of making nanometer fibers comprises the step of providing an electrospinning apparatus.

8. The process according to claim 1, wherein the step of making a skincare product comprises the steps of:
making a film from the nanometers; and
cutting the film into a skincare product in a desired shape.
